# EUROPÄISCHE PATENTSCHRIFT

(11) **EP 2 373 813 B1**
(45) Veröffentlichungstag und Bekanntmachung des Hinweises auf die Patenterteilung: **31.10.2012**
(21) Anmeldenummer: 09805919.9
(22) Anmeldetag: 29.12.2009
(51) Int. Cl.: C12Q 1/68

(54) **VERFAHREN ZUM NACHWEIS METHICILLIN-RESISTENTER STAPHYLOCOCCUS AUREUS (MRSA)-STÄMME**
METHOD FOR DETECTING METHICILLIN-RESISTANT STAPHYLOCOCCUS AUREUS (MRSA) STRAINS
PROCÉDÉ DESTINÉ À DÉTECTER DES SOUCHES DE STAPHYLOCOQUE DORÉ RÉSISTANT À LA MÉTHICILLINE (SARM)

(30) Priorität: 30.12.2008 DE 102008063360
(43) Veröffentlichungstag der Anmeldung: 12.10.2011
(73) Patentinhaber: Qiagen Hamburg GmbH, 22767 Hamburg (DE)
(72) Erfinder: WIEZER, Arnim, 20255 Hamburg (DE)
(74) Vertreter: Roth, Carla
(86) Internationale Anmeldenummer: PCT/EP2009/009302
(87) Internationale Veröffentlichungsnummer: WO 2010/076013

(56) Entgegenhaltungen:
- WO-A2-2008/127839
- WO-A2-2008/140612
- US-A1- 2002 168 697
- US-A1- 2003 180 733
- US-A1- 2004 241 824
- US-A1- 2007 154 903
- SIMEONI ET AL: "Antibiotic resistance genes and identification of staphylococci collected from the production chain of swine meat commodities" FOOD MICROBIOLOGY, ACADEMIC PRESS LTD, LONDON, GB LNKD- DOI:10.1016/J.FM.2007.09.004, Bd. 25, Nr. 1, 8. November 2007 (2007-11-08), Seiten 196-201, XP022336944 ISSN: 0740-0020
- LINDSEY W C ET AL: "Development of a rapid diagnostic assay for methicillin-resistant Staphylococcus aureus and methicillin-resistant coagulase-negative Staphylococcus" DIAGNOSTIC MICROBIOLOGY AND INFECTIOUS DISEASES, ELSEVIER SCIENCE PUBLISHING CO., AMSTERDAM, NL LNKD- DOI:10.1016/J.DIAGMICROBIO.2008.03.002, Bd. 61, Nr. 3, 1. Juli 2008 (2008-07-01), Seiten 273-279, XP022735521 ISSN: 0732-8893 [gefunden am 2008-04-28]
- SABET N S ET AL: "Simultaneous species identification and detection of methicillin resistance in staphylococci using triplex real-time PCR assay" DIAGNOSTIC MICROBIOLOGY AND INFECTIOUS DISEASES, ELSEVIER SCIENCE PUBLISHING CO., AMSTERDAM, NL LNKD- DOI:10.1016/J.DIAGMICROBIO.2006.02.013, Bd. 56, Nr. 1, 1. September 2006 (2006-09-01), Seiten 13-18, XP025146204 ISSN: 0732-8893 [gefunden am 2006-09-01]

## Beschreibung

Die vorliegende Erfindung betrifft ein Verfahren zum Nachweis von MRSA in einer Probe, sowie geeignete Kits und Mittel zur Durchführung entsprechender Verfahren.

### HINTERGRUND DER ERFINDUNG

*Staphylococcus aureus* (*S. aureus*) ist ein Gram-positives Bakterium, das die Haut besiedelt und bei ungefähr 25-30% gesunder Menschen in der vorderen Nasenhöhle vorkommt. Es kann eine Reihe von Krankheiten bei Menschen verursachen, zum Beispiel Wundinfektionen, Lungenentzündung, Sepsis und Endokarditis. Zur Behandlung von *S*. *aureus* Infektionen werden bevorzugt Beta-Lactamantibiotika eingesetzt, wobei Methicillin, Oxacillin, Dicloxacillin und Flucloxacillin Beispiele für solche Beta-Lactamantibiotika sind.

Nach der Einführung von Methicillin in den 60iger Jahren wurde das Auftreten von *S. aureus* Stämmen beobachtet, die gegen Methicillin resistent waren, sogenannte "Methicillinresistente *Staphylococcus aureus*-Stämme" oder kurz "MRSA"-Stämme. MRSA ist seit den 80iger Jahren ein erhebliches klinisches und epidemiologisches Problem in Krankenhäusern, da MRSA gegen sämtliche Beta-Lactamantibiotika, einschließlich Penicillin, Cephalosporin, Carbapenem und Monobactam, die vornehmlich zur Behandlung von *S. aureus* Infektionen eingesetzt werden, resistent ist. MRSA Infektionen können nur mit teureren Antibiotika mit höherer Toxizität behandelt werden, wobei dennoch viele der Fälle für die Betroffenen tödlich enden.

Die Methicillin Resistenz wird durch die Aufnahme und den Einbau eines exogenen Gens, des sogenannten *mecA* Gens, verursacht. Das *mecA* Gen kodiert für ein zusätzliches Beta-Lactam-resistentes Penicillin-Bindeprotein (PBP), das als PBP 2a oder PBP2' bezeichnet wird. Dieses übernimmt die biosynthetischen Funktionen der normalen PBPs, wenn die Zelle Beta-Lactamantibiotika ausgesetzt wird. Das *mecA* Gen ist in MSSA Stämmen ("Methicillinsusceptible *S. aureus")* nicht vorhanden, kommt jedoch als hoch konserviertes Gen bei vielen anderen Arten von Staphylokokken vor, zum Beispiel *Staphylococcus epidermidis, S. haemolyticus, S. saprophyticus, S. capitis, S. warneri, S. sciuri und S. caprae.*

Das *mecA* Gen ist Teil eines großen mobilen genetischen Elements, das als Staphylokokken-Kassetten-Chromosom mec ("Staphylococcus cassette chormosome mec (SCCmec)) bezeichnet wird und von einem Methicillin-anfälligen *S*. *aureus* Stamm (MSSA) aufgenommen wird und diesen dadurch in einen MRSA Stamm umwandelt. Diese Kassette wird in direkter Nähe des bakteriellen Replikationsursprungs (origin of replication) eingebaut.

SCCmec ist durch das Vorhandensein terminal invertierter und direkter Repeats, einem Satz von Stellen-spezifischen Rekombinasegenen und dem mec Genkomplex charakterisiert (Hiramatsu et al., 2002, Int. J. Med. Microbiol. 292:67-74). Die SCCmec DNA wird an einer spezifischen Stelle in das MSSA Chromosom eingebaut, wobei diese Stelle am 3' Ende eines offenen Leserasters (open reading frame ORF) lokalisiert ist, dessen Funktion nicht bekannt ist und das als orfX bezeichnet wird.

Bis heute sind bis zu sieben verschiedene SCCmec Typen und mehrere Varianten davon beschrieben worden. Die verschiedenen SCCmec Elemente unterscheiden sich beispielsweise in den Antibiotikaresistenzgenen gegen Nicht-Beta-Lactamantibiotika.

Da MRSA sehr leicht vom Klinikpersonal auf Patienten übertragen werden kann, ist die Kontrolle von MRSA in Krankenhäusern weltweit ein erhebliches Problem. Daher gibt es einen starken Bedarf nach schnellen und einfachen Screening-Verfahren, mit denen MRSA nachgewiesen, bzw. identifiziert werden kann, um die Ausbreitung zu verringern und die Diagnose bzw. Behandlung der betroffenen Patienten zu verbessern.

Es sind verschiedene Verfahren zum Nachweisen von MRSA vorgeschlagen und auch eingesetzt worden. Die frühen molekularen Tests basierten auf dem Nachweis eines *S.aureus* spezifischen Gens und/oder *mecA.* Der Nachteil dieser Verfahren besteht darin, dass sie nicht für den direkten Nachweis von MRSA aus Proben wie beispielsweise einem Nasenabstrich geeignet sind, da zunächst *S.aureus* Bakterien spezifisch angereichert werden müssen, da die Proben weitere Staphylokokken enthalten können, die ebenfalls *mecA* enthalten.

Ein auf separaten PCRs basierendes Testverfahren weist *mecA* und eine für *S.aureus* spezifische Nukleotidsequenz nach. Aber auch hier besteht das Problem, dass *mecA* in verschiedenen Staphylokokken enthalten ist, so dass zum Beispiel auch Proben als falsch positiv erkannt werden, die MSSA und *S.epidermidis (mecA+)* enthalten. Somit kann der direkte Nachweis des *mecA* Gens in einer Probe nicht als Beweis für das Vorhandensein von MRSA dienen. Dieses Verfahren macht somit nur dann Sinn, wenn zunächst ein Bakterienstamm aus der Probe des Patienten kultiviert und dann als *S.aureus* identifiziert worden ist.

Das Patent EP 0 887 424 offenbart ein Verfahren zum Nachweisen des Vorhandenseins von MRSA, bei dem eine Reaktion mit einer Probe durchgeführt wird, durch kombinierte Verwendung, als Primer und/oder als Sonde, von: (1) einem Teil einer *mecA* DNA, die eine integrierte nicht-erbliche DNA ist, die auf einem Chromosom des MRSA vorliegt und ein mecA-Gen trägt, und (2) einem Teil einer Basensequenz von die integrierte DNA umgebender chromosomaler DNA.

Echt-Zeit PCR (Real time PCR) Ansätze nutzen die SCCmec Insertionsstelle (orfX) und diese umgebende chromosomale DNA zum Nachweis von MRSA. Die äußersten rechten SCCmec Sequenzen (SCCmec right extremity sequences (SRE)) variieren in den verschiedenen SCCmec Typen und grenzen an chromosomale DNA von *S*. *aureus,* nachdem die Kassette in das *S*. *aureus* Genom integriert ist. Bei dem Verfahren, das in der internationalen Patentanmeldung WO 02/099034 offenbart ist, werden spezifische mec-side Primer und MSSA-side Primer in einer PCR Reaktion verwendet, um die Integration der SCCmec Kassette in das MSSA Genom nachzuweisen. Es kommt nur dann zu PCR-Produkten, wenn die Kassette tatsächlich integriert ist. Vor allem kommt es dann nicht zu PCR Produkten, wenn MSSA ohne SCCmec vorliegt bzw. wenn ein anderer Staphylokokken-Stamm vorliegt, der zwar *mecA* enthält, aber nicht MSSA ist. Ein Nachteil dieses Verfahrens liegt darin, dass neue Typen von SCCmec möglicherweise nicht detektiert werden können, nämlich in dem Fall, dass die SRE Sequenzen von den bekannten abweichen, so dass die mec-side Primer nicht mehr binden können und daher kein PCR Produkt gebildet wird, obwohl MRSA vorliegt. Ein weiterer Nachteil liegt darin, dass es in wenigen Fällen vorkommt, dass die SCC Kassette kein *mecA* enthält, so dass es hier zu falsch positiven Signalen kommt, da zwar ein *S.aureus* nachgewiesen wird, der auch eine SCC Kassette enthält, der aber dennoch nicht Methicillin-resistent ist.

Weitere Nachweisverfahren für MRSA werden in WO 2008 140612 A2 sowie in Simeoni et al. (2007), Antibiotic resistance genes and identification of staphylococci collected from the production chain of swine meat commodities, Food Microbiology 25(1), S.196-201 offenbart.

Im Stand der Technik besteht daher Bedarf an weiteren Verfahren zum Nachweis von MRSA. Aufgabe der Erfindung ist es daher, solche bereit zu stellen.

### ÜBERSICHT ÜBER DIE ERFINDUNG

Die vorliegende Erfindung beruht auf der Erkenntnis, dass es zum Nachweis von MRSA in einer Probe vorteilhaft ist, in einem ersten Schritt Target-DNA vom Rest der Probe abzutrennen und dann in einem zweiten Schritt einen Nachweis durchzuführen, der in Kombination mit dem ersten Schritt MRSA spezifisch ist. Die Kombination dieser beiden Schritte erlaubt den effizienten Nachweis von MRSA auch in Mischproben, d.h. in Proben, die neben der Target-DNA auch Non-Target DNA enthalten. Beispiele für Non-Target DNA sind humane DNA oder DNA anderer Organismen, die bspw. nicht MRSA sind.

Gemäß einem ersten Aspekt der vorliegenden Erfindung wird ein Verfahren zum Nachweis von MRSA in einer Probe bereitgestellt. Das erfindungsgemäße Verfahren kann gemäß zwei Varianten durchgeführt werden, die beide auf dem oben genannten Prinzip beruhen.

Gemäß einer ersten Ausführungsform des erfindungsgemäßen Verfahrens (Variante A) wird ein Verfahren zum Nachweis von MRSA in einer Probe zur Verfügung gestellt, bei dem man
a) chromosomale DNA von *S*. *aureus* mittels einer Genomsonde aus der Probe isoliert, und
b) eine Nukleotidsequenz, die spezifisch für MRSA ist, in der isolierten DNA nachweist.

In Schritt a) wird mittels einer Genomsonde chromosomale DNA von *S.aureus* aus der Probe isoliert. In dieser Variante stellt die *S.aureus* DNA die Target-DNA dar. Mittels der Genomsonde kann die *S.aureus* DNA von der Non-Target DNA in der Probe abgetrennt werden. Dadurch wird erreicht, dass chromosomale *S.aureus* DNA spezifisch aus der Probe isoliert bzw. angereichert wird und von der DNA anderer Organismen, die das Testergebnis verfälschen könnten (zum Beispiel *mecA* tragende Staphylokokken wie bspw. *Staphylococcus epidermidis, S. haemolyticus, S. saprophyticus* und *S.capitis,* siehe oben), getrennt wird. In Schritt b) erfolgt dann in der in Schritt a) isolierten Target-DNA der Nachweis einer Nukleotidsequenz, die spezifisch für MRSA ist (bspw. das *mecA* Gen). Dieser spezifische Nachweisschritt ermöglicht die Prüfung, ob in der isolierten *S.aureus* DNA tatsächlich MRSA DNA nachweisbar ist und die analysierte Probe entsprechend MRSA positiv ist. Die Kombination der Schritte a) und b) ermöglicht daher auf effiziente Weise den Nachweis von MRSA in einer Probe.

Gemäß einer zweiten Ausführungsform des erfindungsgemäßen Verfahrens (Variante B) wird ein Verfahren zum Nachweis von MRSA in einer Probe zur Verfügung gestellt, bei dem man
a) DNA aus der Probe isoliert, wobei für die Isolierung eine Genomsonde verwendet wird, die spezifisch für eine Nukleotidsequenz, vorzugsweise ein Resistenzgen von MRSA ist, und
b) die in Schritt a) isolierte DNA auf spezifische Sequenzen von *S*. *aureus* testet.

In Schritt a) wird mittels einer Genomsonde DNA aus der Probe isoliert, die ein Resistenzgen von MRSA aufweist. In dieser Variante stellt die DNA, die eine Nukleotidsequenz (vorzugsweise ein Resistenzgen wie *mecA)* von MRSA aufweist, die Target-DNA dar. Mittels der Genomsonde wird wiederum diese Target-DNA von der Non-Target DNA in der Probe abgetrennt. Eine Non-Target DNA wäre in dieser Variante des erfindungsgemäßen Verfahrens bspw. eine DNA, die kein Resistenzgen von MRSA aufweist (bspw. humane DNA, oder DNA von MSSA Stämmen). Durch die Isolierung mittels der für eine Nukleotidsequenz von MRSA spezifischen Genomsonde wird ebenfalls erreicht, dass die Target-DNA spezifisch aus der Probe isoliert bzw. angereichert wird und von der Non-Target DNA getrennt werden kann. Wie oben ausgeführt gibt es jedoch auch Stämme, die MRSA Sequenzen wie bspw. das *mecA* Gen tragen, jedoch keine *S.aureus* Bakterien und entsprechend nicht MRSA sind. Daher wird gemäß Variante B in Schritt b) getestet, ob die in Schritt a) isolierte DNA *S.aureus* Sequenzen aufweist und die analysierte Probe entsprechend MRSA positiv ist. Zu diesem Zweck wird die in Schritt a) isolierte DNA auf das Vorhandensein von Sequenzen getestet, die spezifisch für *S.aureus* sind. Die Kombination der Schritte a) und b) ermöglicht daher auf effiziente Weise den Nachweis von MRSA in einer Probe.

Gemäß eines weiteren Aspekts der vorliegenden Erfindung wird ein Kit zum Nachweis von MRSA in einer Probe bereitgestellt. Dieses Kit ist für die Durchführung des erfindungsgemäßen Verfahrens geeignet.

Gemäß einer ersten Ausführungsform des Kits (Variante A), wird ein Kit zum Nachweis von MRSA in einer Probe zur Verfügung gestellt, der wenigstens folgende Komponenten enthält:
a) wenigstens eine Genomsonde, die zur Isolierung von DNA von *S. aureus* geeignet ist, und
b) Mittel zum Nachweis einer Nukleotidsequenz, die spezifisch für MRSA ist.

Dieses Kit ist insbesondere zur Durchführung des erfindungsgemäßen Verfahrens gemäß Variante A geeignet. Die in dem erfindungsgemäßen Kit enthaltende Genomsonde ermöglicht die Isolierung der *S.aureus* Target-DNA, was insbesondere bei Mischproben von Vorteil ist. Ferner enthält das Kit Mittel zum Nachweis einer Nukleotidsequenz, die spezifisch für MRSA ist. Einzelheiten hinsichtlich des Zusammenwirkens der entsprechenden Elemente sind bereits oben im Zusammenhang mit dem entsprechenden Verfahren (Variante A) beschrieben; es wird auf die entsprechenden Ausführungen verwiesen. Geeignete Mittel sind dem Fachmann wohlbekannt und sind neben Sonden beispielsweise Oligonukleotide oder Oligonukleotidmimetika, die einen entsprechenden Nachweis mittels PCR ermöglichen.

Gemäß einer weiteren Ausführungsform des Kits (Variante B), wird ein Kit zum Nachweis von MRSA in einer Probe zur Verfügung gestellt, der wenigstens folgende Komponenten enthält:
a) wenigstens eine Genomsonde, die zur Isolierung von eine Nukleotidsequenz, vorzugsweise ein Resistenzgen von MRSA aufweisender DNA aus einer Probe geeignet ist, und
b) Mittel zum Nachweis von *S.aureus* spezifischen DNA - Sequenzen.

Dieses Kit ist insbesondere zur Durchführung des erfindungsgemäßen Verfahrens gemäß Variante B geeignet. Die in dem erfindungsgemäßen Kit enthaltende Genomsonde ermöglicht die Isolierung der Target-DNA, die eine Nukleotidsequenz, vorzugsweise Resistenzgen von MRSA aufweist, was insbesondere bei Mischproben von Vorteil ist. Ferner enthält das Kit Mittel zum Nachweis von *S.aureus* spezifischen DNA - Sequenzen. Einzelheiten hinsichtlich des Zusammenwirkens der entsprechenden Elemente sind bereits oben im Zusammenhang mit dem Verfahren (Variante B) beschrieben; es wird auf die entsprechenden Ausführungen verwiesen. Geeignete Mittel für den Nachweis von DNA-Sequenzen sind dem Fachmann wohlbekannt und sind neben Sonden beispielsweise Oligonukleotide oder Oligonukleotidmimetika, die einen entsprechenden Nachweis mittels PCR ermöglichen.

Weitere Aufgaben, Merkmale, Details und Ausführungsformen der vorliegenden Erfindung ergeben sich aus der nachfolgenden Beschreibung sowie den beigefügten Ansprüchen. Jedoch dient die nachfolgende Beschreibung nur zur Illustration der vorliegenden Erfindung.

### DETAILLIERTE BESCHREIBUNG DER ERFINDUNG

Die der Erfindung zugrundeliegende Aufgabe wird unter anderem durch das erfindungsgemäße Verfahren nach Anspruch 1 gemäß Variante A gelöst.

Die Erfindung betrifft somit gemäß einem Aspekt ein Verfahren zum Nachweis von MRSA in einer Probe, bei dem man:
a) Chromosomale DNA von *S*. *aureus* aus der Probe isoliert, und
b) eine Nukleotidsequenz, die spezifisch für MRSA ist, in der isolierten DNA nachweist.

In einer bevorzugten Ausführungsform handelt es sich bei der Nukleotidsequenz um ein Gen. In einer weiteren bevorzugten Ausführungsform handelt es sich bei dem Gen um ein Resistenzgen, und in einer besonders bevorzugten Ausführungsform handelt es sich bei dem Resistenzgen um *mecA.*

"Spezifisch für MRSA" bedeutet im vorliegenden Fall, dass die Nukleotidsequenz in MRSA aber nicht in MSSA Stämmen vorkommt.

Das erfindungsgemäße Verfahren hat den Vorteil, dass durch die im ersten Schritt durchgeführte Isolierung der *S*. *aureus* DNA sichergestellt ist, dass es sich, falls im zweiten Schritt eine MRSA spezifische Nukleotidsequenz, insbesondere das Resistenzgen *mecA,* nachgewiesen wird, tatsächlich um MRSA handelt. Durch das erfindungsgemäße Verfahren kann MRSA somit spezifisch auch in Mischproben nachgewiesen werden, ohne dass eine Kultivierung der Bakterien notwendig wäre. Das Risiko einer Detektion von Falsch-Positiven, die durch den Nachweis von MSSA und dem *mecA* Gen aus einem anderen Staphylokokken Stamm verursacht wird, wird durch die Kombination der Target-DNA spezifischen Isolation in Schritt a) und dem MRSA spezifischen Nachweis in Schritt b) bei dem erfindungsgemäßen Verfahren somit reduziert. Außerdem ist das erfindungsgemäße Verfahren nicht auf die Verwendung der SRE Sequenzen angewiesen, so dass neue SCCmec Sequenzen mit unbekannten SRE Sequenzen nicht übersehen werden können.

Die chromosomale DNA kann vor oder gleichzeitig mit der Isolierung aus den Bakterienzellen freigesetzt werden. Ein vollständiger Aufschluss der Bakterienzellen ist für die Isolierung der chromosomalen DNA vorteilhaft und daher bevorzugt.

Die chromosomale *S. aureus* DNA kann durch jedes im Stand der Technik bekannte Verfahren isoliert werden. In einer bevorzugten Ausführungsform wird die chromosomale *S. aureus* DNA mittels einer spezifischen Genomsonde isoliert. Bei der Genomsonde handelt es sich um eine Nukleotidsequenz, die zu einem bestimmten Teil der chromosomalen *S. aureus* DNA komplementär ist, wobei dieser Teil spezifisch für *S*. *aureus* ist. Solche für *S. aureus* spezifischen DNA Sequenzen können vom Fachmann ermittelt werden, zum Beispiel durch Genomvergleiche mit Bioinformatikprogrammen.

In einer bevorzugten Ausführungsform ist die Genomsonde zu einem Abschnitt der chromosomalen *S. aureus* DNA komplementär, der in der Nähe der SCCmec Insertionsstelle liegt. Dadurch wird sichergestellt, dass die SCCmec Kassette, falls sie in das Genom integriert ist, was bei MRSA immer der Fall ist, ebenfalls isoliert wird, selbst wenn es bei der Isolierung der chromosomalen *S. aureus* DNA zur Bildung von Fragmenten kommt.

Gemäß einer Ausführungsform wird die *S.aureus* spezifische Genomsonde so ausgewählt, dass sie zu einem Abschnitt der chromosomalen *S.aureus* DNA komplementär ist, der innerhalb eines Bereichs von 20kb, vorzugsweise 10kb, besonders bevorzugt 5kb der SCCmec Insertionsstelle liegt. Je dichter der Abschnitt an den die Genomsonde bindet am SCCmec Insertionsbereich liegt, desto geringer ist das Risiko, dass im Falle der DNA-Fragmentierung während der DNA-Isolierung, die SCCmec Kassette und damit die nachzuweisende MRSA spezifische Nukleotidsequenz nicht (zumindest teilweise) auf dem Fragment liegt, das an die Genomsonde gebunden wird und entsprechend in Schritt b) nachgewiesen werden kann. Durch die Auswahl eines Abschnitts in der Nähe der SCCmec Insertionsstelle wird daher die Wahrscheinlichkeit erhöht, dass auch im Falle der DNA-Fragmentierung die nachzuweisende MRSA spezifische Nukleotidsequenz (sofern in der Probe vorhanden) zumindest teilweise auf einem *S.aureus* DNA-Fragment liegt, das an die Genomsonde gebunden und damit isoliert wurde.

Gemäß einer Ausführungsform wird eine Genomsonde eingesetzt, die zu einem Abschnitt der chromosomalen *S.aureus* DNA komplementär ist, der in der Nähe des *mecA* Gens liegt und *S.aureus* spezifisch ist. Vorzugsweise ist die Genomsonde zu einem Abschnitt komplementär, der innerhalb eines Bereichs von 25kb, vorzugsweise 10kb, besonders bevorzugt 5kb des *mecA* Gens liegt und *S.aureus* spezifisch ist. Diese Variante ist von Vorteil, sofern das *mecA* Gen als MRSA spezifische Nukleotidsequenz nachgewiesen wird. Im Falle der DNA-Fragmentierung während der DNA-Isolierung erhöht sich durch dieses Design die Wahrscheinlichkeit, dass an die Genomsonde ein DNA-Fragment gebunden wird, das (zumindest teilweise) das *mecA* Gen trägt.

Gemäß einer Ausführungsform weist die in dem erfindungsgemäßen Verfahren gemäß Variante A eingesetzte Genomsonde wenigstens folgende Sequenz auf: ATGAAAGCTTTATTACTTAAAACAAGTGTATGGCTCGTTTTGCTTTTTAGTGTAATGGGAT TATGGCAAG (SEQ ID NO 7). Wie die Beispiele zeigen, ist diese Genomsonde geeignet, um chromosomale *S.aureus* DNA aus einer Probe zu isolieren. Damit ist der für diese Genomsonde ausgewählte *S.aureus* Genomabschnitt ein geeignetes Target zur Isolierung von *S.aureus* spezifischer DNA, bei der im Anschluss auch noch Nukleotidsequenzen nachgewiesen werden können, die spezifisch für MRSA sind. Entsprechend kann auch eine *S.aureus* spezifische Genomsonde eingesetzt werden, die zu einem Abschnitt der chromosomalen *S.aureus* DNA komplementär ist, der innerhalb von 1kb, vorzugsweise innerhalb von 500bp, besonders bevorzugt innerhalb von 250bp des Bereiches des *S.aureus* Chromosoms liegt, zu dem die Sequenz mit der SEQ ID NO 7 komplementär ist. Jedoch können auch andere geeignete Sonden anhand der verfügbaren Datenbankeinträgen zu S. *aureus* gestaltet und auf ihre Effizienz hin geprüft werden.

In einer bevorzugten Ausführungsform wird ein Gemisch von verschiedenen Genomsonden zur Isolierung der chromosomalen *S. aureus* DNA eingesetzt. Dies stellt sicher, dass trotz einer möglichen Fragmentbildung der chromosomalen *S. aureus* DNA die entsprechenden Fragmente, die die MRSA spezifische Nukleotidsequenz, insbesondere das MRSA spezifische Resistenzgen, enthalten, isoliert und somit durch das erfindungsgemäße Verfahren nachgewiesen werden können.

Gemäß einer Ausführungsform weist die Genomsonde eine Länge von ≥ 20 Nukleotiden und vorzugsweise ≤ 100 Nukleotiden auf. Dieser Längenbereich hat sich als besonders geeignet erwiesen, um einerseits *S.aureus* DNA spezifisch zu binden, andererseits jedoch ungewünschte Faltungen und Hybridisierungen der Genomsonde zu vermeiden.

Gemäß einer Ausführungsform wird die Genomsonde an einen Träger gebunden. Die zur Isolierung verwendeten Genomsonden können an beliebigen Oberflächen von geeigneten Trägem gebunden sein, wie beispielsweise Magnetic Beads, SpinColumn Filtern, usw. Die Isolierung der DNA und/oder die Bindung der Target-DNA an die Genomsonde erfolgt dann mit Verfahren, die im Stand der Technik gut bekannt sind. Verschiedene Möglichkeiten sind im Stand der Technik bekannt, um Genomsonden an die Oberflächen von Trägermaterialien zu binden. Entsprechend bedarf es diesbezüglich keiner detaillierten Beschreibung, einige Varianten sollen dennoch erwähnt werden. So kann die Genomsonde bspw. über einen Spacer oder Linker, bspw. einen Nukleotidspacer an den Träger gebunden werden. Dies hat sterische Vorteile, insbesondere wenn lange DNA-Fragmente isoliert werden sollen.

Gemäß einer Ausführungsform ist die Genomsonde kovalent an einen Träger gebunden. Diese Ausführungsform ist von Vorteil, wenn die DNA - Aufreinigung aus der biologischen Probe direkt mit der Genomsonde erfolgt und entsprechend kein allgemeiner DNA-Aufreinigungsschritt der Isolierung der Target- DNA in Schritt a) vorgelagert ist.

Ferner können nicht-kovalente Kopplungssysteme zur Anbindung der Genomsonde an den Träger eingesetzt werden (bspw. eine Anbindung über Streptavidin/Biotin). Entsprechende Systeme sind im Stand der Technik wohlbekannt und auch kommerziell verfügbar.

Gemäß einer Ausführungsform wird die Genomsonde in einem ersten Schritt mit der DNA in Kontakt gebracht und in einem zweiten Schritt wird der Träger hinzugegeben, so dass die Genomsonde an den Träger gebunden werden kann. Ein entsprechendes System kann bspw. verwendet werden, wenn die Genomsonde nicht-kovalent an den Träger gebunden ist und wurde auch in dem Beispiel verwendet. Jedoch kann die Genomsonde auch an den Träger gebunden vorliegen, bevor sie mit der DNA in Kontakt gebracht wird.

Für die Isolierung der Nukleinsäure aus der Probe können im Stand der Technik bekannten Verfahren eingesetzt werden. Im Stand der Technik sind bspw. viele Verfahren zur Nukleinsäureaufreinigung bekannt, die aus der Kombination einer festen Phase mit einem chaotropen Puffer bestehen. Ein für eine Vielzahl unterschiedlicher Anwendungen geeignetes Verfahren zur Isolierung von Nukleinsäuren ist beispielsweise in der US 5,234,809 offenbart. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA bindenden festen Phase beschrieben. Die chaotropen Puffer realisieren, sofern notwendig, sowohl die Lyse des Ausgangsmaterials wie auch die Bindung der Nukleinsäuren an die feste Phase. Das Verfahren ist gut geeignet, um Nukleinsäuren aus kleineren Probemengen zu isolieren. Ein auf einem ähnlichen Prinzip beruhendes Verfahren ist auch in WO93/11221 beschrieben. Solche Verfahren zur unspezifischen DNA Aufreinigung können dem erfindungsgemäßen Verfahren vorgeschaltet sein.

Gemäß einer Ausführungsform des erfindungsgemäßen Verfahrens wird in einem vorgelagerten Aufreinigungsschritt die Gesamt-DNA aus der Probe isoliert, bevor gemäß Variante A in Schritt a) die chromosomale *S.aureus* DNA aus der vorgereinigten Gesamt-DNA mittels der Genomsonde isoliert wird. Gemäß einer anderen Ausführungsform des erfindungsgemäßen Verfahrens erfolgt die Isolierung der chromosomalen *S.aureus* DNA aus der biologischen Probe direkt mittels der Genomsonde. Gemäß dieser Ausführungsform ist kein allgemeiner DNA-Aufreinigungsschritt der Isolierung der Target- DNA in Schritt a) vorgelagert. Entsprechendes gilt für das erfindungsgemäße Verfahren gemäß Variante B, das nachfolgend noch im Detail beschrieben wird.

Die Isolierung der chromosomalen *S. aureus* DNA in Schritt a) des erfindungsgemäßen Verfahrens gemäß Variante A erfolgt beispielsweise durch in Kontaktbringen der biologischen Probe mit den Genomsonden, wodurch die chromosomale *S*. *aureus* DNA an diese bindet. Zur Bindung geeignete Bedingungen sind dem Fachmann allgemein bekannt. Wenn beispielsweise Magnetic Beads (magnetische Partikel) verwendet werden, werden diese mit der biologischen Probe in Kontakt gebracht, die gegebenenfalls zuvor oder gleichzeitig so behandelt wird, dass die DNA, die in den Bakterienzellen vorhanden ist, aus diesen freigesetzt wird, wodurch die chromosomale *S*. *aureus* DNA an die Genomsonden an den Beads bindet. Diese können dann mit Hilfe eines Magneten von dem Rest der Probe sowie der ggf. ebenfalls freigesetzten Non-Target DNA separiert, gewaschen und anschließend kann die gebundene chromosomale *S. aureus* DNA von der Genomsonde eluiert bzw. freigesetzt werden. Danach erfolgt der Nachweis der MRSA spezifischen Nukleotidsequenz in Schritt b). Entsprechendes gilt für das erfindungsgemäße Verfahren gemäß Variante B, das nachfolgend noch im Detail beschrieben wird.

In einer bevorzugten Ausführungsform wird ein Resistenzgen als MRSA spezifische Nukleotidsequenz nachgewiesen, und in einer besonders bevorzugten Ausführungsform können mehr als ein MRSA spezifisches Resistenzgen nachgewiesen werden. In einer besonders bevorzugten Ausführungsform ist das Resistenzgen *mecA.*

Die MRSA spezifische Nukleotidsequenz kann mit jedem im Stand der Technik bekannten Nukleinäure-Nachweisverfahren nachgewiesen werden.

In einer bevorzugten Ausführungsform erfolgt der Nachweis der MRSA spezifischen Nukleotidsequenz, insbesondere des Resistenzgens wie bspw. *mecA,* mittels einer PCR. In einer besonders bevorzugten Ausführungsform erfolgt der Nachweis mittels einer Real-Time PCR. Für die PCR bzw. Real-Time PCR geeignete Primer und Detektionssonden können vom Fachmann leicht hergestellt werden. Für diesen Zweck können neben Oligonukleotiden auch Oligonukleotidmimetika wie bspw. PNAs oder LNAs eingesetzt werden. Jedoch sind auch andere Nachweisverfahren denkbar, wie bspw. der Einsatz von markierten Sonden, die die MRSA spezifische Nukleotidsequenz detektieren können. Hierbei kann es sich ebenfalls um Oligonukleotide oder Oligonukleotidmimetika handeln. Entsprechende Nachweisverfahren sind im Stand der Technik wohlbekannt und benötigen daher keine detaillierte Beschreibung. Geeignete Primer sind in dem Beispiel gezeigt (SEQ ID NO 9 bis 11). Wie in dem Beispiel gezeigt, ermöglichen diese den PCR-Nachweis von *mecA.* Die Primer gemäß SEQ ID NO 9 und 10 ermöglichen den regulären PCR-Nachweis, die Sonde gemäß SEC ID NO 11 ermöglicht in Kombination mit den anderen beiden Primern den Nachweis mittels Real-Time PCR.

Die der Erfindung zugrundeliegende Aufgabe wird ferner durch das erfindungsgemäße Verfahren nach Anspruch 1 gemäß Variante B gelöst.

Gemäß dieser weiteren Ausführungsform der Erfindung wird ein Verfahren zum Nachweis von MRSA in einer Probe bereitgestellt, bei dem man
a) DNA aus einer Probe isoliert, wobei für die Isolierung eine Genomsonde verwendet wird, die spezifisch für eine Nukleotidsequenz, vorzugsweise ein Resistenzgen, von MRSA ist, und
b) die in Schritt a) isolierte DNA auf spezifische Sequenzen von *S. aureus* testet.

Dieses Verfahren ermöglicht ebenfalls einen schnellen und spezifischen Nachweis von MRSA in einer Mischprobe. Durch Schritt a) wird zunächst die DNA Isoliert, die Nukleotidsequenzen, insbesondere Resistenzgene aufweist, die auch in MRSA gefunden werden. Bevorzugt handelt es sich dabei um *mecA.* Da neben *S. aureus* noch weitere Staphylokokken MRSA spezifische Nukleotidsequenzen, wie bspw. *mecA,* enthalten, wird auch die DNA dieser, die entsprechende MRSA spezifische Nukleotidsequenz aufweisenden Bakterien im ersten Schritt isoliert, sofern sie in der Mischprobe vorlagen. Ob tatsächlich MRSA vorliegt, kann dann im zweiten Schritt durch den Nachweis von *S. aureus* spezifischen Sequenzen in der in Schritt a) isolierten DNA nachgewiesen werden.

Genau wie in der ersten, oben beschriebenen Ausführungsform des erfindungsgemäßen Verfahrens (Variante A) kann auch in der zweiten Ausführungsform (Variante B) eine Mischung von Genomsonden für die Isolierung der DNA in Schritt a) eingesetzt werden.

Die MRSA Nukleotidsequenz, die in Schritt a) für die Isolierung mittels der Genomsonde genutzt wird (bspw. das *mecA* Gen), kann in unterschiedlichen Bereichen der SCCmec Kassette lokalisiert sein. Ferner kann nicht ausgeschlossen werden, dass aufgrund der zu erwartenden DNA-Fragmentierung während der DNA Isolierung unterschiedlich lange DNA-Fragmente isoliert werden. Daher besteht das Risiko, dass bspw. zwar ein *mecA* aufweisendes MRSA DNA-Fragment in Schritt a) isoliert wird, der *S.aureus* Nachweis in Schritt b) dennoch negativ ausfällt, weil die *S.aureus* spezifische Sequenz, die in Schritt b) nachgewiesen werden soll, bspw. aufgrund der Fragmentierung nicht in dem isolierten MRSA-Fragment enthalten ist. Um dieses Risiko zu senken wird gemäß einer Ausführungsform der Variante B die in Schritt a) isolierte DNA auf mehrere unterschiedliche spezifische Sequenzen von *S.aureus* getestet. Vorzugsweise liegen diese unterschiedlichen *S.aureus* spezifischen Sequenzen beabstandet voneinander, bspw. auf unterschiedlichen Genomabschnitten. Durch den Nachweis mehrerer unterschiedlicher *S.aureus* spezifischer Sequenzen wird das Risiko von falsch negativen Resultaten weiter reduziert.

Gemäß einer Ausführungsform der Variante B des erfindungsgemäßen Verfahrens erfolgt der Test der in Schritt a) isolierten DNA auf *S*. *aureus* spezifische Sequenzen mittels PCR. Details zu einem entsprechenden PCR-Nachweis wurden bereits im Zusammenhang mit dem erfindungsgemäßen Verfahren gemäß Variante A erläutert und sind analog auf die Ausführungsform gemäß Variante B anwendbar. Es wird auf obige Erläuterungen verwiesen.

Einzelheiten zu möglichen Gestaltungen der Genomsonden, Trägern, Oberflächenmaterialien, Anbindung der Genomsonden an den Träger, Verfahren zur Isolierung der DNA und weiteren Ausgestaltungen des erfindungsgemäßen Verfahrens wurden bereits im Zusammenhang mit dem erfindungsgemäßen Verfahrens nach Variante A beschrieben und sind analog auf die Ausführungsform gemäß Variante B anwendbar. Es wird auf obige Erläuterungen verwiesen.

In einer bevorzugten Ausführungsform der erfindungsgemäßen Verfahren wird zusätzlich eine Kontroll-DNA nachgewiesen, die spezifisch für eine humane Sequenz ist, um sicher zu stellen, dass die Probe tatsächlich eine menschliche Probe ist.

Bei der Probe kann es sich um jede Art von biologischer Probe handeln, insbesondere Körperflüssigkeiten. In einer bevorzugten Ausführungsform ist die biologische Probe eine humane Probe und in einer besonders bevorzugten Ausführungsform ist die Probe ein Nasenabstrich eines humanen Patienten. Wie ausgeführt kann die Probe auch eine bereits aufgereinigten DNA sein, die aus einer entsprechenden biologischen Probe gewonnen wurde.

Die vorliegende Erfindung stellt außerdem neue Primer-/ und Sondensequenzen zur Verfügung, die einen Nachweis von MRSA ermöglichen. Hierbei handelt es sich um die folgenden Sequenzen:

| | | |
|---|---|---|
| SauChr | TCAATTAACACAACCCGCATCATTTG | (SEQ ID NO:1) |
| saur-sd | Fam-CGCATAATCTTAAATGCTCTATACACTTG-BHQ1 | (SEQ ID NO:2) |
| type5aw-rev | CACTAGTGTAATTATCGAATGATTTATAACTAC | (SEQ ID NO:3) |
| pvl_for | TTACACAGTTAAATATGAAGTGAACTGG | (SEQ ID NO:4) |
| pvl_rev | CTGCATCAACTGTATTGGATAGC | (SEQ ID NO:5) |
| pvl_sd | Hex-AAACTCATGAAATTAAAGTGAAAGGACATAATTGA-BHQ 1 | (SEQ ID NO:6) |

Diese Primer können unter anderem in den oben beschriebenen Real-Time PCR Verfahren vorteilhaft eingesetzt werden.

Es gibt besonders aggressive MRSA Stämme, die unter anderem dadurch gekennzeichnet sind, dass sie das sogenannte Panton-Valentine-Leukocidin (PVL bzw. PVL Toxin) aufweisen. Zum Nachweis solcher MRSA Stämme sind bevorzugt die Sequenzen mit den SEQ ID NOs:4, 5 und 6 einsetzbar.

In einer weiteren Ausführungsform betrifft die vorliegende Erfindung somit ein Verfahren zum Nachweis von MRSA Stämmen, bei dem man:
1. Chromosomale DNA von *S. aureus* aus der Probe isoliert;
2. eine Nukleotidsequenz, die spezifisch für MRSA ist, in der isolierten DNA nachweist, und
3. das Vorhandensein von PVL nachweist.

In einer bevorzugten Ausführungsform handelt es sich bei der Nukleotidsequenz um ein Resistenzgen, und in einer besonders bevorzugten Ausführungsform handelt es sich bei dem Resistenzgen um *mecA.*

Eine weitere Ausführungsform der vorliegenden Erfindung betrifft ein Verfahren, bei dem man:
1. DNA aus einer Probe isoliert, wobei für die Isolierung eine Genomsonde verwendet wird, die spezifisch für eine Nukleotidsequenz, vorzugsweise ein Resistenzgen, von MRSA ist;
2. die in Schritt 1 isolierte DNA auf spezifische Sequenzen von S. *aureus* testet, und
3. das Vorhandensein von PVL nachweist.

In einer bevorzugten Ausführungsform handelt es sich bei dem Resistenzgen um *mecA.*

Diese Verfahren zum Nachweis von MRSA haben den Vorteil, dass sie zusätzlich das Vorhandensein des PVL Toxins nachweisen, welches einen besonders schweren Krankheitsverlauf verursacht.

Der Nachweis von PVL erfolgt bevorzugt mittels der Sequenzen der SEQ ID NOs: 4, 5 und 6 im Rahmen einer Real-Time PCR.

Ein weiteres erfindungsgemäßes Verfahren ist durch den Einsatz einer Multi-Locus PCR definiert. Bei diesem Verfahren werden mehrere Loci mittels PCR nachgewiesen, die in der Kombination einen Nachweis von MRSA ermöglichen. Geeignete Loci sind bevorzugt *mecA* und ein *S.aureus* Locus. Die PCRs können als separate PCRs oder als Multiplex PCR durchgeführt werden.

Ferner wird mit der vorliegenden Erfindung ein Kit zum Nachweis von MRSA in einer Probe zur Verfügung gestellt, der wenigstens folgende Komponenten enthält:
Variante A
   a) wenigstens eine Genomsonde, die zur Isolierung von DNA von *S. aureus* geeignet ist, und
   b) Mittel zum Nachweis einer Nukleotidsequenz, die spezifisch für MRSA ist;
   oder
Variante B
   a) wenigstens eine Genomsonde, die zur Isolierung von eine Nukleotidsequenz, vorzugsweise ein Resistenzgen, von MRSA aufweisender DNA aus einer Probe geeignet ist, und
   b) Mittel zum Nachweis von *S.aureus* spezifischen DNA- Sequenzen.

Dieses Kit ist insbesondere zur Durchführung der oben beschriebenen erfindungsgemäßen Verfahren geeignet. Die in dem erfindungsgemäßen Kit enthaltende Genomsonde ermöglicht die Isolierung der Target-DNA. Ferner enthält das Kit Mittel zum Nachweis einer MRSA spezifischen Nukleotidsequenz (Variante A) oder Mittel zum Nachweis einer *S.aureus* spezifischen Nukleotidsequenz. Geeignete Mittel zum Nachweis spezifischer Sequenzen sind dem Fachmann wohlbekannt und sind neben markierter Sonden beispielsweise Oligonukleotide oder Oligonukleotidmimetika, die einen entsprechenden Nachweis mittels PCR ermöglichen.

Die in dem erfindungsgemäßen Kit enthaltende Genomsonde gemäß Variante A ermöglicht die Isolierung der *S.aureus* Target-DNA, was insbesondere bei Mischproben von Vorteil ist. Ferner enthält das Kit Mittel zum Nachweis einer Nukleotidsequenz, die spezifisch für MRSA ist. Einzelheiten hinsichtlich des Zusammenwirkens der entsprechenden Elemente sind bereits oben im Zusammenhang mit dem entsprechenden erfindungsgemäßen Verfahren (Variante A) beschrieben; es wird auf die entsprechenden Ausführungen verwiesen.

Gemäß einer Ausführungsform weist das Kit gemäß Variante A eine Genomsonde auf, die zu einem Abschnitt der chromosomalen *S. aureus* DNA komplementär ist, wobei der Abschnitt spezifisch für *S*. *aureus* ist. Dies ermöglicht die spezifische Isolierung von *S.aureus* DNA aus einer Probe, die neben *S.aureus* DNA auch andere Non-Target DNA enthält.

Gemäß einer Ausführungsform weist das Kit gemäß Variante A eine Genomsonde auf, die zu einem Abschnitt der chromosomalen *S.aureus* DNA komplementär ist, der in der Nähe der SCCmec Insertionsstelle liegt. Vorzugsweise weist das Kit eine Genomsonde auf, die zu einem Bereich innerhalb von 20kb, vorzugsweise 10kb, besonders bevorzugt 5kb der SCCmec Insertionsstelle komplementär ist. Gemäß einer bevorzugten Ausgestaltung enthält das Kit eine Genomsonde, die die Sequenz mit der SEQ ID NO 7 aufweist. Das Kit kann ferner eine Genomsonde aufweisen, die innerhalb von 1kb, vorzugsweise 500bp, besonders bevorzugt 250bp des Bereiches von *S.aureus* bindet, zu dem die Sequenz mit der SEQ ID NO 7 komplementär ist. Die Vorteile einer entsprechenden Ausgestaltung wurden bereits oben im Zusammenhang mit dem erfindungsgemäßen Verfahren gemäß Variante A erläutert.

Vorzugsweise weist das Kit gemäß Variante A Mittel zum PCR-Nachweis der Nukleotidsequenz, die spezifisch für MRSA ist, vorzugsweise *mecA,* auf. Als Mittel zum PCR-Nachweis von *mecA* kann das Kit wenigstens einen Primer aufweisen, der eine Sequenz aufweist, die ausgewählt ist aus den Sequenzen mit der SEQ ID NO 9 bis 11. Wie in dem Beispiel gezeigt, ermöglichen diese den PCR-Nachweis von *mecA.* Die Primer gemäß SEQ ID NO 9 und 10 ermöglichen den regulären PCR-Nachweis, die Sonde gemäß SEQ ID NO 11 ermöglicht in Kombination mit den anderen beiden Primern den Nachweis mittels Real-Time PCR.

Die in dem erfindungsgemäßen Kit gemäß Variante B enthaltende Genomsonde ermöglicht die Isolierung der Target-DNA, die eine MRSA spezifische Nukleotidsequenz, vorzugsweise ein Resistenzgen wie *mecA,* aufweist, was insbesondere bei Mischproben von Vorteil ist. Ferner enthält das Kit Mittel zum Nachweis von *S.aureus* spezifischen DNA - Sequenzen. Einzelheiten hinsichtlich des Zusammenwirkens der entsprechenden Elemente sind bereits oben im Zusammenhang mit dem Verfahren (Variante B) beschrieben; es wird auf die entsprechenden Ausführungen verwiesen.

Gemäß einer Ausführungsform weist das Kit gemäß Variante B Mittel zum Nachweis mehrerer unterschiedlicher spezifischer Sequenzen von *S.aureus* auf. Vorzugsweise liegen diese unterschiedlichen *S.aureus* spezifischen Sequenzen beabstandet voneinander, bspw. auf unterschiedlichen Genomabschnitten. Durch den Nachweis mehrerer unterschiedlicher *S.aureus* spezifischer Sequenzen wird das Risiko von falsch negativen Resultaten weiter reduziert, wie oben im Zusammenhang mit dem erfindungsgemäßen Verfahren gemäß Variante B erläutert. Es wird auf die obige Offenbarung verwiesen.

Vorzugsweise weist das Kit gemäß Variante B Mittel zum PCR-Nachweis der *S. aureus* spezifischen Sequenzen auf.

Gemäß einer Ausführungsform weist das Kit eine Genomsonde auf, die eine Länge von ≥ 20 Nukleotiden und vorzugsweise ≤ 100 Nukleotiden aufweist.

Das Kit kann ferner mehrere unterschiedliche Genomsonden aufweisen. Vorteile wurden im Zusammenhang mit den erfindungsgemäßen Verfahren erläutert, es wird auf obige Ausführungen verwiesen.

Gemäß einer bevorzugten Ausführungsform weist das Kit einen Träger zur Anbindung der Genomsonde auf. Die Genomsonde kann ferner an den Träger gebunden vorliegen. Einzelheiten und Vorteile wurden im Zusammenhang mit den erfindungsgemäßen Verfahren erläutert, es wird auf obige Ausführungen verwiesen.

Vorzugsweise weist das Kit zum Nachweis der spezifischen Sequenzen Oligonukleotide oder Oligonukleotidmimetika auf. Einzelheiten und Vorteile hierzu wurden im Zusammenhang mit den erfindungsgemäßen Verfahren erläutert, es wird auf obige Ausführungen verwiesen.

### BEISPIEL

Das erfindungsgemäße Verfahren gemäß Variante A wird anhand des folgenden Ausführungsbeispiels illustriert. Dieses beschreibt lediglich eine mögliche Ausführungsform der Erfindung und ist daher nicht beschränkend. Analoge Verfahren können für die Durchführung des Verfahrens gemäß Variante B eingesetzt werden.

### I. Material

Folgende Materialien wurden eingesetzt:
1. Dynabeads kilobaseBINDER Kit (Invitrogen Kat. Nr. 601.01).
2. Eine Genomsonde zur Isolierung von chromosomaler DNA von *S.aureus* mit nachfolgender Sequenz: Der klein geschriebene Bereich am Anfang der Sonde ist artifiziell und dient lediglich als Spacer zum Biotin und damit später zum Träger, an den die Genomsonde gebunden wird. Die *S.aureus* spezifische Sequenz der Genomsonde wurde anhand des folgenden Datenbankeintrages designt:
   LOCUS CP000255 2319 bp DNA linear BCT 12-MAR-2009
   DEFINITION Staphylococcus aureus subsp. aureus USA300_FPR3757, complete genome.
   ACCESSION CP000255 REGION: 31026..33344
3. Ein Dynal Magnet
4. Ein Vortexer
5. Pipetten
6. Roller
7. NaOH 0,125 M (frisch!) - "Schmeizlösung"
8. 20 % Essigsäure

### II. Aufreinigung der S.aureus spezifischen DNA

Der Versuch wurde anhand des folgenden Protokolls durchgeführt:
1. Die Dynabeads werden durch schütteln oder vortexen des Fläschchen resuspendiert, um eine homogene Suspension zu erhalten.
2. 5 µl (50 µg) der resuspendierten Beads werden in ein 1,5 ml Mikrozentrifugen-Röhrchen überführt. Die Röhrchen werden 2 Minuten über dem Magnet platziert (oder so lange, bis alle Beads an den Rand gewandert sind).
3. Der Überstand wird vorsichtig abpipettiert, während das Röhrchen auf dem Magnet verbleibt. Eine Berührung der Beadpellets mit der Pipettenspitze sollte vermieden werden.
4. Das Röhrchen vom Magneten entfernen. 20 µl Binding-Solution (Bestandteil des Dynabeads kilobaseBINDER Kits) werden an der Innenseite des Röhrchens, wo sich die Beads gesammelt haben, zugegeben und die Beads werden behutsam mit der Pipette resuspendiert. Die Lösung kann viskos sein.
5. Die Röhrchen werden wieder auf dem Magnet platziert und der Überstand (Binding Solution) wird wie in Schritt 3 oben entfernt.
6. Die Beads werden in 20 µl Binding-Solution resuspendiert.
7. 350 pmol (70 pmol pro 1 µg Beads) der Genomsonde (siehe oben) werden zu 10⁵ Genomkopien von *S*. *aureus* geben. Hierfür wurden 20 µl einer entsprechenden *S. aureus* DNA enthaltenden Probe eingesetzt, Verdünnung: 1:100. In diesem Schritt lagert sich die Genomsonde an die DNA. Für die Zwecke dieses Versuchs wurde aufgereinigte *S.aureus* DNA eingesetzt, um zu zeigen, dass die Genomsonden *S.aureus* DNA binden und isolieren können.
8. Inkubation des Röhrchens bei Raumtemperatur (ca. 15-25 °C) für 1 Stunde auf einem Roller, damit die Beads in Suspension bleiben.
9. 20 µl der Bead Suspension werden zu der Probe gegeben.
10. Inkubation des Röhrchens bei Raumtemperatur (15-25 °C) für 1 Stunde (20 Min) auf einem Roller, um die Beads in Suspension zu halten. In diesem Schritt binden die Beads an das Biotin der Genomsonde. Dadurch erfolgt eine Anbindung der Genomsonde (mit der gebundenen *S.aureus* spezifischen DNA) an die Beads.
11. Die Röhrchen werden auf den Magnet platziert und der Überstand wie in Schritt 3 beschrieben, entfernt.
12. Der Dynabeads/DNA-Komplex wird zweimal in 40 µl der washing solution (Bestandteil des Dynabeads kilobaseBINDER Kits) gewaschen und einmal in destillierten H₂O oder Tris-HCl pH 8.0.
13. Wasser/Puffer komplett entfernen.
14. Vorbereitung der Neutralisationslösung durch Mischen von 500 µl PB und 3,8 µl 20% Essigsäure.
15. 50 µl der "Schmelzlösung" (0,125 M NaOH) zu den Beads geben.
16. Vortexen
17. Überstand durch Magnetischen Partikel Konzentrator (MPC) entfernen.
18. Der Überstand wird in ein Röhrchen mit Neutralisationslösung gegeben.
19. Schritte 15 bis 18 werden wiederholt.
20. Die geschmolzene Probe wird in ein Spin Column überführt, abzentrifugiert und der Durchfluss wird verworfen. 750µl PE wird zugefügt und noch mal runtergeschleudert. Der Durchfluss wird verworfen, das Röhrchen um 180° gedreht und noch mal abzentrifugiert. Die DNA wird mit 15 µl TE-Puffer eluiert.

### III. Nachweis des mecA Resistenzgens

Folgende Real-time PCR wurde durchgeführt, um das mecA Resistenzgen in der Probe und damit MRSA in der zuvor isolierten *S.aureus* DNA nachzuweisen.

Die PCR wurde wie folgt angesetzt:

| **Komponente** | **Konzentration** | **Endkonzentration** | **Menge/ Reaktion** | |
|---|---|---|---|---|
| | | | | |
| Real time PCR Buffer | 5X | 1X | 5 | µl |
| dNTPs | 10 mM | 0,14 mM | 0.35 | µl |
| BSA | 20 mg/ml | 0,2 mg/ml | 0.25 | µl |
| HotStarTaq | 5U/µl | 0,192 U/µl | 0.96 | µl |
| MpCl2 | 200mM | 5mM | 0.625 | µl |
| mec_for (21) | 100µM | 0,5µM | 0.125 | µl |
| mec_rev (22) | 100µM | 0,5µM | 0.125 | µl |
| mec_sd2 (54) | 100µM | 0,05µm | 0.0125 | µl |
| H2O | | | 12.5525 | µl |
| Template | | | 5.00 | µl |
| | | | **25** | µl |

Cycler
Program

| | | | | |
|---|---|---|---|---|
| 95°C | 15 Minuten | | Green | mec-Primer |
| 95°C | 20 Sekunden | | Orange | PVL |
| 53°C | 20 Sekunden | | Crimson | Nuc |
| 72°C | 30 Sekunden | | | |
| | | | | |
| 45 Cycles | | | | |

Für den Nachweis des *mecA* Resistenzgens mittels Realtime PCR wurden folgende Primer/Proben verwendet:

| | |
|---|---|
| mec_for | ATTACCGTTCTCATATAGCTCATCATAC (SEQ ID NO 9) |
| mec_rev | ATAAAGATAATCCAAACATGATGATGGC (SEQ ID NO 10) |
| mec_sd2 | FAM-CCATTCCTTTATCTTGTACATCTTTAACATT-BHQ1 (SEQ ID NO 11) |

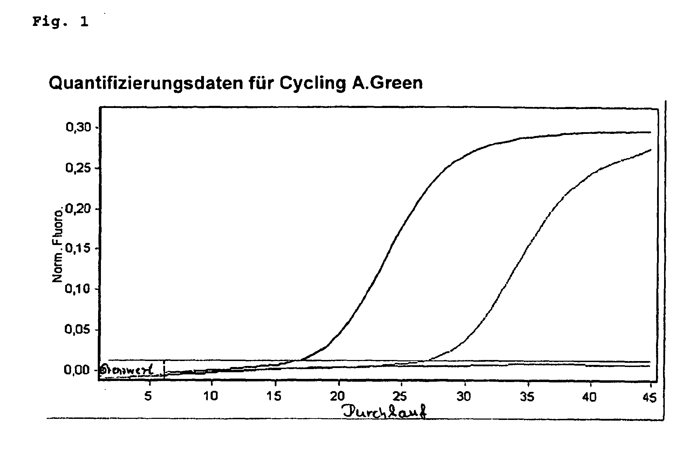

Die Ergebnisse der PCR sind in Fig. 1 gezeigt. Links ist die Kurve gezeigt, bei der die ursprüngliche Probe, d.h. die mittels eines herkömmlichen Verfahrens aufgereinigte *S.aureus* DNA als Template eingesetzt wurde (es erfolgte entsprechend keine spezifische Isolierung mittels der Genomsonde). Rechts ist die Kurve gezeigt, bei der die gemäß dem oben beschriebenen Verfahren unter Einsatz der Genomsonde aufgereinigte *S.aureus* DNA als Template eingesetzt wurde. Die Ergebnisse zeigen, dass mittels der Genomsonden *S.aureus* isoliert werden kann und auch *mecA* nachgewiesen werden konnte. Entsprechend wäre das Verfahren auch geeignet, S.aureus aus einer Mischprobe, in der neben S.aureus DNA noch andere, non-target -DNAs vorliegen, zu isolieren.

## Patentansprüche

1. Verfahren zum Nachweis von MRSA in einer Probe, bei dem man eine der folgenden Verfahrensvarianten durchführt:
Variante A
a) chromosomal DNA von *S. aureus* mittels einer *S. aureus* spezifischen Genomsonde aus der Probe isoliert, und
b) eine Nukleotidsequenz, die spezifisch für MRSA ist, in der isolierten DNA nachweist, wobei die MRSA spezifische Nukleotidsequenz in MRSA aber nicht in MSSA Stämmen vorkommt;
oder
Variante B
a) DNA aus der Probe isoliert, wobei für die Isolierung eine Genomsonde verwendet wird, die spezifisch für eine Nukleotidsequenz von MRSA, vorzugsweise ein Resistenzgen von MRSA, ist, und
b) die in Schritt a) isolierte DNA auf spezifische Sequenzen von *S. aureus* testet.

2. Verfahren nach Anspruch 1, **dadurch gekennzeichnet, dass** die Isolierung der chromosomalen DNA von *S. aureus* gemäß Variante A mit Hilfe einer zu einem Abschnitt der chromosomalen DNA komplementären Nukleinsäure (Genomsonde) isoliert wird, wobei der Abschnitt spezifisch für *S. aureus* ist.

3. Verfahren nach Anspruch 1 oder 2 gemäß Variante A, **dadurch gekennzeichnet, dass** es eines oder mehrere der folgenden Merkmale aufweist:
a) dass die Genomsonde zu einem Abschnitt der chromosomalen *S.aureus* DNA komplementär ist, der in der Nähe der SCCmec Insertionsstelle liegt;
b) dass die Genomsonde zu einem Abschnitt der chromosomalen *S.aureus* DNA komplementär ist, der innerhalb eines Bereichs von 25kb, vorzugsweise 10kb, besonders bevorzugt 5kb der SCCmec Insertionsstelle liegt;
c) dass die Genomsonde zu einem Abschnitt der chromosomalen *S.aureus* DNA komplementär ist, der in der Nähe des *mecA* Gens liegt und *S.aureus* spezifisch ist;
d) dass die Genomsonde zu einem Abschnitt der chromosomalen *S.aureus* DNA komplementär ist, der innerhalb eines Bereichs von 25kb, vorzugsweise 10kb, besonders bevorzugt 5kb des *mecA* Gens liegt uns *S.aureus* spezifisch ist; und/oder
e) dass die Genomsonde folgende Sequenz aufweist: ATGAAAGCTTTATTACTTAAAACAAGTGTATGGCTCGTTTTGCTTTTTAGT GTAATGGGATTATGGCAAG (SEQ ID NO 7) oder eine Genomsonde ist, die zu einem Abschnitt der chromosomalen *S.aureus* DNA komplementär ist, der innerhalb von 1kb, vorzugsweise innerhalb von 500bp, besonders bevorzugt innerhalb von 250bp des Bereiches des *S.aureus* Chromosoms liegt, zu dem die Sequenz mit der SEQ ID NO 7 komplementär ist.

4. Verfahren nach einem oder mehreren der Ansprüche 1 bis 3; **gekennzeichnet durch** eines oder mehrere der folgenden Merkmale:
a) dass die Genomsonde eine Länge von ≥ 20 Nukleotiden und vorzugsweise ≤ 100 Nukleotiden aufweist;
b) dass die Genomsonde an einen Träger gebunden wird;
c) dass die Genomsonde über einen Spacer an den Träger gebunden wird;
d) dass die Genomsonde kovalent an einen Träger gebunden vorliegt;
e) dass die Genomsonde in einem ersten Schritt mit der DNA in Kontakt gebracht wird und in einem zweiten Schritt der Träger hinzugegeben wird;
f) dass die Genomsonde an den Träger gebunden vorliegt, bevor sie mit der DNA in Kontakt gebracht wird; und/oder
g) dass gemäß Variante A mehrere verschiedene Genomsonden zur Isolierung der chromosomalen DNA von *S. aureus* verwendet werden oder gemäß Variante B eine Mischung von Genomsonden für die Isolierung der DNA eingesetzt werden.

5. Verfahren nach einem oder mehreren der Ansprüche 1 bis 4 gemäß Variante A, **dadurch gekennzeichnet, dass** die Nukleotidsequenz ein Resistenzgen ist, wobei das Resistenzgen vorzugsweise *mecA* ist.

6. Verfahren gemäß einem oder mehreren der Ansprüche 1 bis 5, **dadurch gekennzeichnet, dass** gemäß Variante A der Nachweis der Nukleotidsequenz, die spezifisch für MRSA ist, mittels PCR erfolgt oder dass gemäß Variante B der Test der in Schritt a) isolierten DNA auf *S. aureus* spezifische Sequenzen mittels PCR erfolgt.

7. Verfahren gemäß einem oder mehreren der Ansprüche 1 bis 6, **dadurch gekennzeichnet, dass** gemäß Variante B die in Schritt a) isolierte DNA auf mehrere unterschiedliche spezifische Sequenzen von *S.aureus* getestet wird.

8. Verfahren gemäß einem oder mehreren der Ansprüche 1 bis 7, **dadurch gekennzeichnet, dass** die DNA in einem Vorschritt aus der Probe isoliert wird bevor gemäß Variante A in Schritt a) die chromosomale *S.aureus* DNA mittels der Genomsonde isoliert wird oder gemäß Variante B in Schritt b) die eine Nukleotidsequenz von MRSA tragende DNA mittels der Genomsonde isoliert wird.

9. Verfahren nach einem oder mehreren der Ansprüche 1 bis 8, **dadurch gekennzeichnet, dass** es sich bei der Probe um eine Mischprobe handelt.

10. Verfahren nach einem oder mehreren der Ansprüche 1 bis 9, **dadurch gekennzeichnet, dass** ferner das Vorhandensein von Panton-Valentine-Leukocidin (PVL) nachgewiesen wird, vorzugsweise unter Einsatz wenigstens einer der Sequenzen mit den SEQ ID NOs: 4, 5 und 6.

11. Verfahren gemäß einem oder mehreren der Ansprüche 1 bis 10, **dadurch gekennzeichnet, dass** eine Kontroll-DNA nachgewiesen wird, die spezifisch für eine humane Sequenz ist.

12. Verwendung eines Kits zum Nachweis von MRSA in einer Probe zur Durchführung eines Verfahrens gemäß einem oder mehreren der Ansprüche 1 bis 11, wobei das Kit wenigstens folgende Komponenten enthält:
Variante A
a) wenigstens eine *S. aureus* spezifische Genomsonde, die zur Isolierung von DNA von *S. aureus* geeignet ist, und
b) Mittel zum Nachweis einer Nukleotidsequenz, die spezifisch für MRSA ist;
oder
Variante B
a) wenigstens eine Genomsonde, die zur Isolierung von ein Resistenzgen von MRSA aufweisender DNA aus einer Probe geeignet ist, und
b) Mittel zum Nachweis von *S.aureus* spezifischen DNA - Sequenzen.

13. Verwendung eines Kits nach Anspruch 12 Variante A, wobei das Kit **gekennzeichnet ist durch** eines oder mehrere der folgenden Merkmale:
a) dass das Kit eine Genomsonde aufweist, die zu einem Abschnitt der chromosomalen *S. aureus* DNA komplementär ist, wobei der Abschnitt spezifisch für *S. aureus* ist;
b) dass das Kit eine Genomsonde aufweist, die zu einem Abschnitt der chromosomalen *S.aureus* DNA komplementär ist, der in der Nähe der SCCmec Insertionsstelle liegt;
c) dass das Kit eine Genomsonde aufweist, die zu einem Bereich innerhalb von 20kb, vorzugsweise 10kb, besonders bevorzugt 5kb der SCCmec Insertionsstelle komplementär ist;
d) dass das Kit eine Genomsonde mit der Sequenz mit der SEQ ID NO 7 aufweist oder eine Genomsonde aufweist, die innerhalb von 1kb, vorzugsweise 500bp, besonders bevorzugt 250bp des Bereiches von *S.aureus* bindet, zu dem die Sequenz mit der SEQ ID NO 7 komplementär ist;
e) dass das Kit Mittel zum PCR-Nachweis der Nukleotidsequenz, die spezifisch für MRSA ist, vorzugsweise mecA, aufweist; und/oder
f) dass das Kit als Mittel zum PCR-Nachweis von *mecA* wenigstens einen Primer aufweist, der eine Sequenz aufweist, die ausgewählt ist aus den Sequenzen mit der SEQ ID NO 9 bis 11.

14. Verwendung eines Kits nach Anspruch 12 Variante B, wobei das Kit **gekennzeichnet ist durch** eines oder mehrere der folgenden Merkmale:
a) Mittel zum Nachweis mehrerer unterschiedlicher spezifischer Sequenzen von *S.aureus* aufweist; und/oder
b) Mittel zum PCR-Nachweis der *S*. *aureus* spezifische Sequenzen aufweist.

15. Verwendung eines Kits nach einem der Ansprüche 12 bis 14, das Kit **gekennzeichnet durch** eines oder mehrere der nachfolgenden Merkmale:
a) dass das Kit eine Genomsonde aufweist, die eine Länge von ≥ 20 Nukleotiden und vorzugsweise ≤ 100 Nukleotiden aufweist;
b) dass das Kit mehrere unterschiedliche Genomsonden aufweist;
c) dass das Kit einen Träger zur Anbindung der Genomsonde aufweist oder die Genomsonde an den Träger gebunden vorliegt; und/oder
d) dass das Kit zum Nachweis der spezifischen Sequenzen Oligonukleotide oder Oligonukleotidmimetika aufweist.

## Claims

1. Method of detecting MRSA in a sample, wherein one of the following method variants is carried out:
variant A
a) chromosomal DNA of *S. aureus* is isolated from the sample by means of an S. aureus-specific genome probe, and
b) a nucleotide sequence which is specific for MRSA is detected in the isolated DNA, wherein the MRSA-specific nucleotide sequence occurs in MRSA but not in MSSA strains;
or
variant B
a) DNA is isolated from the sample, wherein for isolation a genome probe is used which is specific for an MRSA nucleotide sequence, preferably an MRSA resistance gene, and
b) the DNA isolated in step a) is tested for specific sequences of S. aureus.

2. Method according to claim 1, **characterized in that** the isolation of the chromosomal DNA of *S. aureus* as per variant A is performed using a nucleic acid (genome probe) complementary to a segment of the chromosomal DNA, wherein the segment is specific for *S. aureus*.

3. Method according to claim 1 or 2 as per variant A, **characterized in that** it comprises one or more of the following features:
a) the genome probe is complementary to a segment of the chromosomal *S. aureus* DNA which is close to the SCCmec insertion site;
b) the genome probe is complementary to a segment of the chromosomal *S. aureus* DNA which is located within a region of 25 kb, preferably 10 kb, particularly preferably 5 kb, to the SCCmec insertion site;
c) the genome probe is complementary to a segment of the chromosomal *S. aureus* DNA which is close to the *mecA* gene and is *S.* aureus-specific;
d) the genome probe is complementary to a segment of the chromosomal S. aureus DNA which is located within a region of 25 kb, preferably 10 kb, particularly preferably 5 kb, of the *mecA* gene and is *S. aureus-*specific; and/or
e) the genome probe comprises the following sequence:
ATGAAAGCTTTATTACTTAAAACAAGTGTATGGCTCGTTTTGCTT TTTAGTGTAATGGGATTATGGCAAG (SEQ ID NO 7) or is a genome probe which is complementary to a segment of the chromosomal S. aureus DNA which is located within 1 kb, preferably within 500 bp, particularly preferably within 250 bp, of the region of the S. aureus chromosome to which the sequence with SEQ ID NO 7 is complementary.

4. Method according to one or more of the claims 1 to 3, **characterized by** one or more of the following features:
a) the genome probe is ≥ 20 nucleotides and preferably ≤ 100 nucleotides in length;
b) the genome probe is bound to a support;
c) the genome probe is bound to the support via a spacer;
d) the genome probe is present covalently bonded to a support;
e) the genome probe is contacted with the DNA in a first step and the support is added in a second step;
f) the genome probe is present bound to the support before it is contacted with the DNA; and/or
g) as per variant A, multiple different genome probes are used to isolate the chromosomal DNA of *S. aureus,* or as per variant B, a mixture of genome probes are used to isolate the DNA.

5. Method according to one or more of the claims 1 to 4 as per variant A, **characterized in that** the nucleotide sequence is a resistance gene, wherein the resistance gene is preferably mecA.

6. Method according to one or more of the claims 1 to 5, **characterized in that**, as per variant A, the nucleotide sequence which is specific for MRSA is detected by means of PCR, or **in that**, as per variant B, the DNA isolated in step a) is tested for *S. aureus*-specific sequences by means of PCR.

7. Method according to one or more of the claims 1 to 6, **characterized in that**, as per variant B, the DNA isolated in step a) is tested for multiple different specific sequences of *S. aureus.*

8. Method according to one or more of the claims 1 to 7, **characterized in that** the DNA is isolated from the sample in a preliminary step before, as per variant A, the chromosomal *S. aureus* DNA is isolated by means of the genome probe in step a), or as per variant B, the DNA carrying an MRSA nucleotide sequence is isolated by means of the genome probe in step b).

9. Method according to one or more of the claims 1 to 8, **characterized in that** the sample is a mixed sample.

10. Method according to one or more of the claims 1 to 9, **characterized in that**, in addition, the presence of Panton-Valentine Leukocidin (PVL) is detected, preferably using at least one of the sequences with the SEQ ID NOs: 4, 5 and 6.

11. Method according to one or more of the claims 1 to 10, **characterized in that** a control DNA is detected which is specific for a human sequence.

12. Use of a kit for detecting MRSA in a sample for carrying out a method according to one or more of the claims 1 to 11, wherein the kit comprises at least the following components:
variant A
a) at least one *S. aureus*-specific genome probe which is suitable for isolating DNA of *S. aureus,* and
b) agents for detecting a nucleotide sequence which is specific for MRSA;
or
variant B
a) at least one genome probe which is suitable for isolating, from a sample, DNA having an MRSA resistance gene, and
b) agents for detecting *S. aureus*-specific DNA sequences.

13. Use of a kit according to claim 12 variant A, wherein the kit is **characterized by** one or more of the following features:
a) the kit comprises a genome probe which is complementary to a segment of the chromosomal *S. aureus* DNA, wherein the segment is specific for *S. aureus*;
b) the kit comprises a genome probe which is complementary to a segment of the chromosomal *S. aureus* DNA which is close to the SCCmec insertion site;
c) the kit comprises a genome probe which is complementary to a region located within 20 kb, preferably 10 kb, particularly preferably 5 kb, of the SCCmec insertion site;
d) the kit comprises a genome probe with the sequence with SEQ ID NO 7, or comprises a genome probe which binds within 1 kb, preferably 500 bp, particularly preferably 250 bp, of the region of *S. aureus* to which the sequence with SEQ ID NO 7 is complementary;
e) the kit comprises agents for PCR detection of the nucleotide sequence which is specific for MRSA, preferably *mecA*; and/or
f) the kit comprises, as agents for PCR detection of *mecA,* at least one primer which comprises a sequence selected from the sequences with SEQ ID NO 9 to 11.

14. Use of a kit according to claim 12 variant B, wherein the kit is **characterized by** one or more of the following features:
a) agents for detecting multiple different specific sequences of *S. aureus*; and/or
b) agents for PCR detection of the *S. aureus-*specific sequences.

15. Use of a kit according to any of the claims 12 to 14, the kit being **characterized by** one or more of the following features:
a) the kit comprises a genome probe which is ≥ 20 nucleotides and preferably ≤ 100 nucleotides in length;
b) the kit comprises multiple different genome probes;
c) the kit comprises a support for binding the genome probe or the genome probe is present bound to the support; and/or
d) the kit comprises oligonucleotides or oligonucleotide mimetics for detecting the specific sequences.

## Revendications

1. Procédé de détection du MRSA dans un échantillon, dans lequel on réalise l'une des variantes de procédé suivantes :
Variante A
a) on isole de l'échantillon un ADN chromosomique de *S. aureus* au moyen d'une sonde génomique spécifique de *S. aureus,* et
b) on détecte dans l'ADN isolé une séquence nucléotidique qui est spécifique du MRSA, la séquence nucléotidique spécifique de MRSA existant dans les souches de MRSA mais pas dans les souches de MSSA ;
ou
Variante B
a) on isole de l'échantillon de l'ADN, où on utilise pour l'isolement une sonde génomique qui est spécifique d'une séquence nucléotidique du MRSA, de préférence d'un gène de résistance de MRSA, et
b) on teste l'ADN isolé à l'étape a) pour ce qui est des séquences spécifiques de *S. aureus.*

2. Procédé selon la revendication 1, **caractérisé en ce que** l'isolement de l'ADN chromosomique de *S. aureus* selon la variante A est isolé à l'aide d'un acide, nucléique (sonde génomique) complémentaire d'un segment de l'ADN chromosomique, le segment étant spécifique de *S. aureus.*

3. Procédé selon la revendication 1 ou 2 selon la variante A, **caractérisé en ce qu'**il présente une ou plusieurs des caractéristiques suivantes :
a) **en ce que** la sonde génomique est complémentaire d'un segment de l'ADN chromosomique de *S. aureus* qui se trouve près du site d'insertion SCCmec ;
b) **en ce que** la sonde génomique est complémentaire d'un segment de l'ADN chromosomique de *S. aureus* qui se trouve au sein d'une plage de 25 kb, de préférence de 10 kb, de façon particulièrement préférée de 5 kb, par rapport au site d'insertion SCCmec ;
c) **en ce que** la sonde génomique est complémentaire d'un segment de l'ADN chromosomique de *S. aureus* qui se trouve près du gène *mecA* et est spécifique de *S. aureus ;*
d) **en ce que** la sonde génomique est complémentaire d'un segment de l'ADN chromosomique de *S. aureus* qui se trouve au sein d'une plage de 25 kb, de préférence de 10 kb, de façon particulièrement préféré de 5 kb, par rapport au gène *mecA* et est spécifique de *S. aureus ;* et/ou
e) **en ce que** la sonde génomique présente la séquence suivante :
ATGAAAGCTTTATTACTTAAAACAAGTGTATGGCTCGTTTTGCT TTTTAGTGTAATGGGATTATGGCAAG (ID SEQ. N° 7), ou est une sonde génomique qui est complémentaire d'un segment de l'ADN chromosomique de *S. aureus,* qui se trouve au sein de 1 kb, de préférence au sein de 500 pb, de façon particulièrement préférée au sein de 250 pb de la plage du chromosome de *S. aureus* dont la séquence ayant l'ID SEQ. N° 7 est complémentaire.

4. Procédé selon une ou plusieurs des revendications 1 à 3, **caractérisé par** une ou plusieurs des caractéristiques suivantes :
a) en ce que la sonde génomique présente une longueur ≥ 20 nucléotides et de préférence ≤ 100 nucléotides ;
b) en ce que la sonde génomique est liée à un support ;
c) en ce que la sonde génomique est liée au support par une séquence d'espacement ;
d) en ce que la sonde génomique se présente liée de façon covalente à un support ;
e) en ce que la sonde génomique est mise en contact avec l'ADN lors d'une première étape et en ce que le support est ajouté lors d'une seconde étape ;
f) en ce que la sonde génomique se présente liée au support avant d'être mise en contact avec l'ADN ; et/ou
g) en ce que selon la variante A sont utilisées plusieurs sondes génomiques différentes pour l'isolement de l'ADN chromosomique de *S. aureus* ou selon la variante B est utilisé un mélange de sondes génomiques pour l'isolement de l'ADN.

5. Procédé selon une ou plusieurs des revendications 1 à 4 selon la variante A, **caractérisé en ce que** la séquence nucléotidique est un gène de résistance, le gène de résistance étant de préférence *mecA.*

6. Procédé selon une ou plusieurs des revendications 1 à 5, **caractérisé en ce que** selon la variante A, la détection de la séquence nucléotidique qui est spécifique du MRSA se fait au moyen d'une PCR, ou **en ce que** selon la variante B, le test de l'ADN isolé à l'étape a) portant sur les séquences spécifiques de *S. aureus* se fait au moyen d'une PCR.

7. Procédé selon une ou plusieurs des revendications 1 à 6, **caractérisé en ce que** selon la variante B, l'ADN isolé à l'étape a) est testé pour ce qui est de plusieurs séquences de *S. aureus* différentes et spécifiques.

8. Procédé selon une ou plusieurs des revendications 1 à 7, **caractérisé en ce que** l'ADN est isolé de l'échantillon lors d'une étape préalable avant qu'à l'étape a) selon la variante A, l'ADN chromosomique de *S. aureus* soit isolé au moyen de la sonde génomique ou que selon la variante B à l'étape b), l'ADN portant une séquence nucléotidique de MRSA soit isolé au moyen de la sonde génomique.

9. Procédé selon une ou plusieurs des revendications 1 à 8, **caractérisé en ce qu'**il s'agit concernant la sonde d'une sonde mixte.

10. Procédé selon une ou plusieurs des revendications 1 à 9, **caractérisé en ce que** l'on détecte en outre la présence de leucocidine de Panton Valentine (PVL), de préférence en utilisant au moins une des séquences ayant les ID SEQ. n°4,5 et 6.

11. Procédé selon une ou plusieurs des revendications 1 à 10, **caractérisé en ce que** l'on détecte un ADN de contrôle qui est spécifique d'une séquence humaine.

12. Utilisation d'un kit de détection du MRSA dans un échantillon pour réaliser un procédé selon une ou plusieurs des revendications 1 à 11, dans laquelle le kit contient au moins un des composants suivants :
Variante A
a) au moins une sonde génomique spécifique de *S. aureus* et qui est appropriée pour isoler de l'ADN de *S. aureus,* et
b) des moyens de détection d'une séquence nucléotidique qui est spécifique du MRSA;
ou
variante B
a) au moins une sonde génomique qui est appropriée pour l'isolement dans un échantillon d'ADN présentant un gène de résistance du MRSA, et
b) des moyens pour détecter les séquences d'ADN spécifiques de *S. aureus.*

13. Utilisation d'un kit selon la revendication 12 variante A, dans laquelle le kit est **caractérisé par** une ou plusieurs des caractéristiques suivantes :
a) en ce que le kit présente une sonde génomique qui est complémentaire d'un segment de l'ADN chromosomique de *S. aureus,* le segment étant spécifique de *S. aureus ;*
b) en ce que le kit présente une sonde génomique qui est complémentaire d'un segment de l'ADN chromosomique de *S. aureus* qui se trouve près du site d'insertion SCCmec ;
c) en ce que le kit présente une sonde génomique qui est complémentaire d'une plage qui se trouve au sein de 20 kb, de préférence de 10 kb, de façon particulièrement préférée de 5 kb, par rapport au site d'insertion SSCmec ;
d) en ce que le kit présente une sonde génomique ayant l'ID SEQ n° 7 ou présente une sonde génomique qui se lie au sein de 1 kb, de préférence de 500 pb, de façon particulièrement préférée de 250 pb, de la plage de *S. aureus* dont la séquence ayant l'ID SEQ n° 7 est complémentaire ;
e) en ce que le kit présente des moyens de détection par PCR de la séquence nucléotidique qui est spécifique du MRSA, de préférence de *mecA ;* et/ou
f) en ce que le kit présente en tant que moyen de détection par PCR de *mecA* au moins une amorce qui présente une séquence sélectionnée parmi les séquences ayant les ID SEQ n° 9 à 11.

14. Utilisation d'un kit selon la revendication 12 variante B, dans laquelle le kit est **caractérisé par** une ou plusieurs des caractéristiques suivantes :
a) il présente des moyens de détection de plusieurs séquences différentes et spécifiques de *S. aureus ;* et/ou
b) il présente des moyens de détection par PCR de séquences spécifiques de *S. aureus.*

15. Utilisation d'un kit selon une ou plusieurs des revendications 12 à 14, le kit étant **caractérisé par** une ou plusieurs des caractéristiques suivantes :
a) en ce que le kit présente une sonde génomique qui présente une longueur 20 nucléotides et de préférence ≤ 100 nucléotides ;
b) en ce que le kit présente plusieurs sondes génomiques différentes ;
c) en ce que le kit présente un support pour lier la sonde génomique ou que la sonde génomique se présente liée au support ; et/ou
d) en ce que le kit présente pour la détection des séquences spécifiques des oligonucléotides ou des mimétiques d'oligonucléotides.


### SEQUENCE LISTING

<110> Qiagen GmbH wiezer, Arnim
<120> verfahren zum Nachweis Methicillin-resistenter staphylococcus aureus (MRSA)-Stämme
<130> 51 140 K
<150> DE 10 2008 063 360.7
   <151> 2008-12-30
<160> 11
<170> PatentIn version 3.3
<210> 1
   <211> 26
   <212> DNA
   <213> Artificial
<220>
   <223> Primer SauChr
<400> 1
   tcaattaaca caacccgcat catttg 26
<210> 2
   <211> 29
   <212> DNA
   <213> Artificial
<220>
   <223> Primer probe saur-sd
<220>
   <221> modified_base
   <222> (1)..(1)
   <223> modified with FAM, a fluorescence dye
<220>
   <221> modified_base
   <222> (29)..(29)
   <223> modified with BHQ1, a quencher
<400> 2
   cgcataatct taaatgctct atacacttg 29
<210> 3
   <211> 33
   <212> DNA
   <213> Artificial
<220>
   <223> Primer type5aw-rev
<400> 3
   cactagtgta attatcgaat gatttataac tac 33
<210> 4
   <211> 28
   <212> DNA
   <213> Artificial
<220>
   <223> Primer pvl_for
<400> 4
   ttacacagtt aaatatgaag tgaactgg 28
<210> 5
   <211> 23
   <212> DNA
   <213> Artificial
<220>
   <223> Primer pvl_rev
<400> 5
   ctgcatcaac tgtattggat agc 23
<210> 6
   <211> 35
   <212> DNA
   <213> Artificial
<220>
   <223> Primer probe pvl_sd
<220>
   <221> modified_base
   <222> (1)..(1)
   <223> modified with Hex, a fluorescence dye
<220>
   <221> modified_base
   <222> (35)..(35)
   <223> modified with BHQ1, a quencher
<400> 6
   aaactcatga aattaaagtg aaaggacata attga 35
<210> 7
   <211> 70
   <212> DNA
   <213> Artificial
<220>
   <223> Genome probe for S.aureus
<400> 7
   atgaaagctt tattacttaa aacaagtgta tggctcgttt tgctttttag tgtaatggga 60
   ttatggcaag 70
<210> 8
   <211> 87
   <212> DNA
   <213> Artificial
<220>
   <223> Genome probe for S. aureus
<220>
   <221> misc_feature
   <222> (1)..(1)
   <223> biotinylated
<220>
   <221> misc_feature
   <222> (1)..(17)
   <223> an artificial spacer sequence
<400> 8
   tatcctatcc tatcctgatg aaagctttat tacttaaaac aagtgtatgg ctcgttttgc 60
   tttttagtgt aatgggatta tggcaag 87
<210> 9
   <211> 28
   <212> DNA
   <213> Artificial
<220>
   <223> Primer mec_for, for detection of the mecA gene detection
<400> 9
   attaccgttc tcatatagct catcatac 28
<210> 10
   <211> 28
   <212> DNA
   <213> Artificial
<220>
   <223> Primer mec_rev, for detection of the mecA gene detection
<400> 10
   ataaagataa tccaaacatg atgatggc 28
<210> 11
   <211> 31
   <212> DNA
   <213> Artificial
<220>
   <223> Primer probe mec_sd2 for real-time PCR for the detection of the mecA gene
<220>
   <221> modified_base
   <222> (1)..(1)
   <223> modified with FAM, a fluorescence dye
<220>
   <221> modified_base
   <222> (31)..(31)
   <223> modified with BHQ1, a quencher
<400> 11
   ccattccttt atcttgtaca tctttaacat t 31